Europäisches Patentamt

European Patent Office

Office européen des brevets

(11) Publication number: **0 478 021 A1**

# EUROPEAN PATENT APPLICATION

(21) Application number: **91201717.5**

(22) Date of filing: **03.07.91**

(51) Int. Cl.5: **C12Q 1/32**

(30) Priority: **16.09.90 NL 9002033**

(43) Date of publication of application:
**01.04.92 Bulletin 92/14**

(84) Designated Contracting States:
**AT BE CH DE DK ES FR GB GR IT LI LU NL SE**

(71) Applicant: **Royal Sluis Koninklijke Zaaizaadbedrijven Gebroeders Sluis B.V. Westeinde 161 NL-1601 BM Enkhuizen(NL)**

(72) Inventor: **Van den Berg, Bartel Marinus Sebastiaan Centenweg 45 NL-1602 ML Enkhuizen(NL)**

(74) Representative: **Kupecz, Arpad et al Octrooibureau Los en Stigter B.V. Postbox 20052 NL-1000 HB Amsterdam(NL)**

(54) Method of determining the hybrid purity of F1-hybrids of tomatoes.

(57) The present invention relates to a method of determining the hybrid purity of F1-hybrids of tomatoes by separation of the allelic variants $ADH-1^+$ and $ADH-1^1$ followed by colouring for alcohol dehydrogenase activity and visual detection. According to the present process the separation of $ADH-1^+$ and $ADH-1^1$ is carried out by isoelectric focussing.

fig.1

The present invention relates to a method of determining the hybrid purity of F1-hybrids of tomatoes by separation of the allelic variants ADH-1$^+$ and ADH-1$^1$ followed by colouring for alcohol dehydrogenase activity and visual detection.

The hybrid purity of F1-hybrids of tomatoes is an essential characteristic of seed lots which is of particular importance for the seed company. During the last ten, twenty years the use of hybrid seeds has assumed enormous proportions. In respect of the seeds of open pollinated varieties the seeds of F1-hybrid varieties offer particular advantages. This is related to the manner of development and production of hybrid varieties. In the simplest case one starts from two different parental lines (the male parent which furnishes the pollen and the female line which is pollinated and from which finally the seed is harvested) which are obtained by recombination, selection and inbreeding. By the process of inbreeding or self-pollination the parents of hybrids are homozygous for most properties. Because both parents used for producing hybrids, are so homogeneous by process of self-pollination, the seed to be finally harvested also will be very homogenous of genetic composition. The final crop growing from the hybrid seed is particularly uniform as to properties like morphology, yield, harvest time, manner of culture etc. Besides uniformity hybrid varieties also more often give a surplus yield in respect of pollinated varieties. These advantages of the hybrid varieties for the buyer of the hybrid seeds are of great importance (for example mechanical harvesting is made possible by the uniformity of the crop). This also shows the importance of the hybrid quality of the crop. When the mother line of the hybrid by whatever cause is self-pollinated, this leads to mother seeds instead of hybrid seeds. Also self-pollination of the father line may occur and by circumstances seeds from the father can be harvested. Self-pollination of the mother line leads to inbred seeds in the hybrid seeds. This is only to a very slight degree acceptable for the buyer of the hybrid seeds. Therefore the hybrid quality of seeds in practice is determined by samples taken at random. This consists in sowing a certain number of seeds, permitting the plants to grow under the right conditions to adult fruit bearing stage and subsequently carrying out a detailed analysis of all properties of the hybrid plants. Since the hybrid is the "sum" of both parents and the parents in most cases differ in properties such analysis will yield a reliable result, but this method is time-consuming and expensive. Obtaining data relating to the hybrid quality of the seeds will be long, (some months) to expect and the seeds cannot be sold directly after harvesting. This means that the seeds in many cases have to be stored for a long time, with all the accompanying costs. This not only causes extra costs by storing per se, but also quality loss, missing sales possibilities and a more difficult planning of next productions.

A method described in the preamble is known from Hort. Science 16 (2) pages 179-181, 1981 (Application of Alcohol Dehydrogenase Allozymes in Testing the Genetic Purity of F1 Hybrids of Tomato). In this known method the separation of the allelic variants ADH-1$^+$ and ADH-1$^1$ is established by starch gel electrophoresis. Furthermore, the extract of the imbibed tomato seeds is brought on a strip of filter paper serving as carrier for the extract. Thereafter the filter-paper is placed in a slot of the gel, the filter-paper being perpendicular to the gel. This procedure is very time-consuming and economically unfavourable.

Another disadvantage of starch gel electrophoresis technique is the cumbersome preparation of the starch gel and furthermore the reproducibility of the starch gel leaves much to be desired since the quality of the starting starch is not constant.

Furthermore, due to the relatively large thickness of the starch gel layer of about 0,5 mm the proof of the alcohol dehydrogenase takes a rather long time.

The invention has the object of providing a method which avoids the above-mentioned disadvantages.

The invention provides a method of determining the hybrid purity of F1 hybrids of tomatoes by separation of the allelic variants ADH-1$^+$ and ADH-1$^1$ followed by colouring for alcohol dehydrogenase activity and visual detection, characterized in that the separation of ADH-1$^+$ and ADH-1$^1$ is carried out by isoelectric focusing.

Experimentally it is established that ADH-1$^+$ and ADH-1$^1$ have an isoelectric point of respectively 5,5 and 5,7.

In the method of the invention the seed extract is brought directly on the gel by a pipette without using filter-paper. This is realized by using a flexible application strip of preferably silicone rubber provided with regularly spaced openings. This strip is laid on the gel layer next to the positive electrode whereafter the seed extract is brought by pipette in the individual openings in such a manner that in each opening the extract of a different seed is received.

It is advantageous in the present method that the gel layer used only has a small thickness of about 0,1 mm whereby the determination of the alcohol dehydrogenase bands is possible within 1 to 2 minutes.

Another advantage is that in the isoelectric focussing a gel is used that is prepared in a simple manner from polyacrylamide. Such a gel is well reproducible and may be kept in a refrigerator for a long time in contrast to the starch gels.

Finally, it has surprisingly appeared that in the present method a person may determine the hybrid purity of more than 1500 seeds daily, whereas in the known method only 500 seeds can be examined. It is evident that the invention provides a quick and economical instrument for determining the hybrid purity of tomato seed lots.

It is noted that the technique of isoelectric focussing for determining the hybrid purity of seeds is known from the article by R.J. Cooke and S.R. Draper in J. Natn. Inst. agric. Bot. (1983), 16, 173-181. In this article a method is described for the use of ultra-thin-layer isoelectric focussing for characterizing seeds wherein the individual seeds are crushed whereafter the crushed seeds or portion thereof are extracted in a small volume (100-250 $\mu$l) 1,0 M ureum with or without 2 volume percent 2.mercapto ethanol (dependent on the varieties) in a polypropylene centrifuge tube.

After eventual degreasing the ureum solution is treated with Amberlite MB-1 whereafter the finally obtained clear supernatant is used for focussing.

Besides seeds like those of wheat, oats lucerne and rape seed also the seed proteins of peas and beans can be determined.

A usual technique is using ampholytes for creating the pH-gradient in the gel whereby the resolution of the proteins is promoted.

According to this known article the known method has the advantage of obtaining an excellent resolution of the seed proteins in a much quicker manner than in the case of the conventional gel electrophoresis techniques.

This article is silent about the use of such isoelectric focussing method for determining the hybrid purity of tomato seeds.

In general the procedure of the present method comprises transferring tomato seeds to microtiter plates, i.e. one seed at a time in one cavity. Then the cavities are filled with water. After incubation during the night (16-20 hours) in order to induce de novo synthesis of ADH-1 under anaerobic conditions the water is removed whereafter in each of the cavities 80 $\mu$l fresh water is introduced. Then the seeds are homogenized by a home-made device attached to a drilling machine. Of the so obtained homogenate a quantity of 7 $\mu$l is transferred by a pipette to the gel surface using a sample applicator strip. This strip has a number of openings and is located at about 5 mm distance from the anode whereafter isoelectric focussing is carried out as follows.

A Desaga electrophoresis device (DESAPHOR HE 200) is used which is provided with a cooled plate of 200 x 265 x 1 mm and is connected to a Pharmacia constant current supply ECPS 3000/150 and a Lauda RC3 cooling device (at a temperature of 10°C). The gels were laid on the cooling plate with a few drops of kerosine to ensure good contact. The electrode strips were soaked in 0,5 M aspartic acid and 0,5 M glutamic acid (anode strips) and cathode liquid 10 (cathode strips). The electrodes were placed on the strips and thereafter covered by a glass plate as weight to ensure good electric contact. Three electrodes were placed on the gel in case the gel was divided in two parts for the analysis of 2 x 96 seeds. Prefocussing was carried out in an arrangement of 10 W, 1500 V and 17 mA. The prefocussing begins at about 280 V until the voltage reached about 720 V (150 Vh). After applying the samples the focussing was continued at the same arrangements for 2000 Vh (50-60 min). In case the gel was partitioned in three parts for the analysis of 3 x 96 seeds four electrodes were placed on the gel. Then the prefocussing was carried out at an arrangement of 7 W, 1250 V and 30 mA. The prefocussing started at about 180 V until the voltage increased to about 400 V (15) Vh). After applying the sample the focussing was continued at 5 VH at 50 V, 7 W, 1250 V, then prefocussing during 1 hour and 15 min (1500 Vh).

The colouring for ADH-activity directly after isoelectric focussing was realized as follows.

The gel was incubated in a solution of 0,1 M Tris-HCl pH 7,5 with therein 4% alcohol. After 5 min., when the coloured bands were visible, the gel was washed during 1 min. with a solution of ethanol (40%), water (50%) and acetic acid (10%) to avoid background colouring. After flushing with water the gels were dried in the air and stored.

Isoelectric focussing produces in the gel electrophoretic patterns, which are interpreted as follows. One examines by the pattern which seeds are F1 hybrid pure and which are not. In the F1 hybrid the characteristic bands of both mother and father may be refound. When in the reference pattern the father-specific bands fails, the seed is not genetically pure. Assuming that a hundred seeds are examined which are carried along on the isoelectric focussing patterns, then for tomatoes all hundred have to be pure for a good seed quality for a certain lot. Even if one single seed is not pure, such lot is less desired. In general the seed price is primarily determined by the degree of hybrid quality. The desired degree of hybrid quality and the relation between price is different from crop to crop. For tomatoes, for example, very high demands are put on the hybrid purity.

The invention is now explained by the following figures wherein

Fig. 1 represents an isoelectric focussing pattern for tomato F1 hybrid Arletta and

Fig. 2 represents a schematic illustration of the hybrid quality determination or determination of inbreeding of seeds by isoelectric focussing.

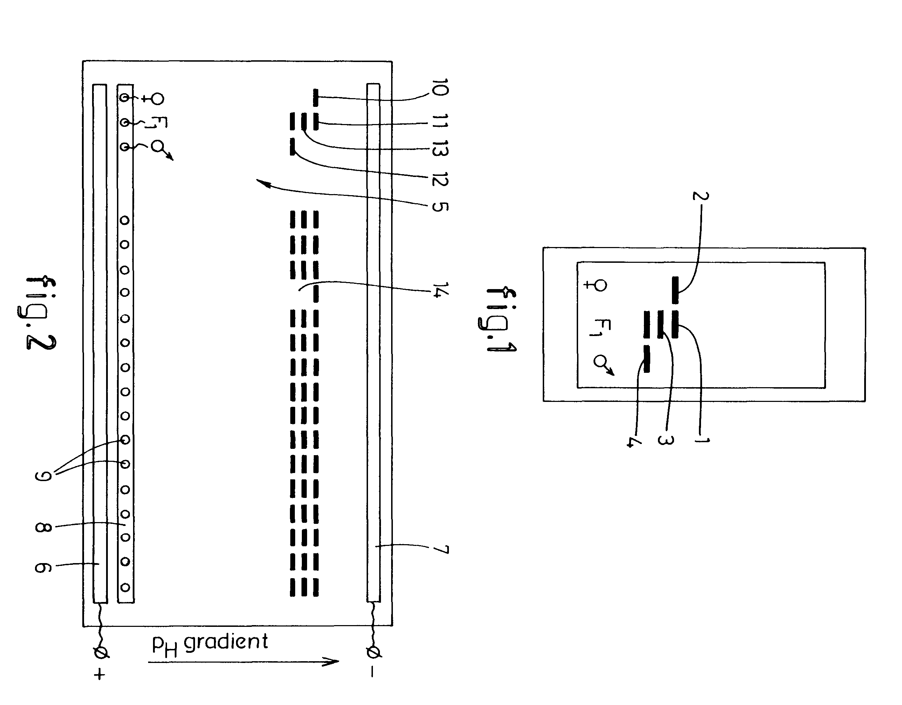

Fig. 1 represents the F1 hybrid pattern obtained by isoelectric focussing for the tomato Arletta wherein the band for the father-specific enzyme 4 as well as the band for the mother-specific enzyme 2 are shown.

Fig. 2 represents the determination of the hybrid purity also called inbreeding determination, of a lot of tomato seeds by isoelectric focussing in a schematic way. A gel plate 5 is used provided with a positive electrode 6 and a negative electrode 7. Just above the positive electrode at about 5 mm there is located a sample application strip 8 of rubber provided with cavities 9. Furthermore, mother-specific respectively father-specific enzyme bands 10 and 12 are represented as well as a F1 hybrid pattern 11 wherein both father-and mother-specific enzyme bands are present.

In the determination of inbreeding of a lot of tomato seeds an aqueous extract of one seed at a time from the lot of seeds is introduced into one cavity 9 of the sample application strip 8.

Then one takes an extract of a seed wherein only mother-specific enzymes are present and of a seed wherein only father-specific enzymes are present and finally, one of a F1 hybrid wherein both kinds of enzymes are present. When applying the desired potential difference between the positive and negative electrodes 6 and 7 one obtains at a certain pH gradient and after colouring with a suitable colouring agent the enzyme pattern represented in Fig. 2. In the first three cavities from left to right are respectively introduced the mother-specific, F1 hybrid and father-specific enzyme solutions and in the other cavities the enzyme solutions of the seeds of which the hybrid purity, i.e. the degree of inbreeding is to be determined. The mother- and father-specific enzymes also are called marker enzymes. Should one of the marker enzymes fail, as for example at 14 (at 14 fails the father-specific enzyme) then there is a question of inbreeding.

In this manner the hybrid purity of F1 hybrid tomato seeds may be determined.

In the method of the invention commonly water is used that was purified by the Milli-Q system (Millipore, Bedford, USA).

Furthermore are used: Acrylamide, ammonium-persulphate, ampholytes (servalyt pH 3-10), aspartic acid, bis, cathode liquid 10 (on the basis of arginine, lysine and ethylene diamine), Coomassie Brilliant Blue R-250 (Serva Blue R), glutamic acid, kerosine from Serva (Heidelberg, Federal Republic of Germany); glycine, TCA, pI protein kit (range 4,75-10,4) from BDH (Poole, United Kingdom). Acetic acid, NADP, NBT, PMS and other chemicals not mentioned here were from Sigma Chem. Co. (St. Louis, USA).

The gel fixation used in the invention for coatings and gel fixation for PAGE (265 x 193 mm) and electrode strips (300 x 6 x 1 mm) are from Serva (Heidelberg, Federal Republic of Germany). Flat bottom microtiter plates with 96 cavities are from Costar (Cambridge, USA). Adhesive tape (tesa, 100 $\mu$m) was from Beun de Ronde (Abcoude, NL). Silicone rubber application strips were made from 1 mm thick silicone rubber sheets (Eriks, Alkmaar, NL).

The ultra-thin-layer isoelectric focussing of the invention, abbreviated UTLIEF, is a known method proceeding as follows. Firstly gels are prepared.

UTLIEF gels were prepared by the "flap" technique as described by Radola. Adhesive tape is used as distance tape along each side of the gel fixation film (265 mm x 193 mm). Using three electrodes two gels of the dimension 250 x 90 mm could be operated simultaneously. The gel fixation and coating films are rolled out on glass plates with a few drops between the glass plate and the film. The polymerization mixture contained 1,68 ml acrylamide (40% T + 5% C), 0,6 ml servalyt pH 3-10, 5,72 ml 16% glycerol and 0,4 ml 1,5% ammonium persulphate. The final acrylamide concentration was 8%. This mixture was strongly degassed on a vacuum conduit during 1-2 min. After "flapping" and polymerization the gels were directly used (after at least 1 hour) or stored at 4°C during maximally one week.

**Claims**

1. A method of determining the hybrid purity of F1-hybrids of tomatoes by separation of the allelic variants ADH-1$^+$ and ADH-1$^1$ followed by colouring for alcohol dehydrogenase activity and visual detection, **characterized** in that the separation of ADH-1$^+$ and ADH-1$^1$ is carried out by isoelectric focussing.

fig.1

fig.2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X,P | ELECTROPHORESIS vol. 12, no. 1, 1 January 1991, WEINHEIM BRD pages 64 - 69; B.M. VAN DEN BERG.: 'A rapid and economical method for hybrid purity testing of tomato (lycopersicon esculentum L) F1 hybrids using ultrathin-layer isoelectric focusing of alcohol dehydrogenase variants from seeds.' * See whole article* * | 1 | C 12 Q 1/32 |
| Y,D | HORTSCIENCE vol. 16, no. 2, 2 April 1981, USA pages 179 - 181; S.D. TANKSLEY ET AL: 'Application of alcohol de-hydrgenase alloenzymes in testing the genetic purity of f1 hybrids of tomato.' * See whole article* * | 1 | |
| Y,D | JOURNAL OF THE NATIONAL INSTITUTE FOR AGRICULTURAL BOTANY vol. 16, 1983, USA pages 173 - 181; R.J. COOKE ET AL: 'Potential applications of ulta-thin layer isoelectric focusing for the characterisation of cultivars of crop species' * See whole article* * | 1 | |
| T | ELECTROPHORESIS vol. 11, no. 10, 1 October 1990, WEINHEIM BRD pages 824 - 829; B.M.VAN DEN BERG .: 'Inbred testing of tomato (Lycopersicon esculentum)' * See whole article* * | 1 | **TECHNICAL FIELDS SEARCHED (Int. Cl.5)** <br><br> C 12 Q |

The present search report has been drawn up for all claims

| Place of search | Date of completion of search | Examiner |
|---|---|---|
| The Hague | 07 January 92 | VAN BOHEMEN C.G. |

CATEGORY OF CITED DOCUMENTS
X : particularly relevant if taken alone
Y : particularly relevant if combined with another
    document of the same catagory
A : technological background
O : non-written disclosure
P : intermediate document
T : theory or principle underlying the invention

E : earlier patent document, but published on, or after
    the filing date
D : document cited in the application
L : document cited for other reasons

&  : member of the same patent family, corresponding
    document